# EUROPEAN PATENT APPLICATION

(11) **EP 4 079 241 A1**
(43) Date of publication of application: **26.10.2022**
(21) Application number: 22168129.9
(22) Date of filing: 13.04.2022
(51) Int. Cl.: A61B 17/72

(54) **BONE FIXATION DEVICE**

(30) Priority: 23.04.2021 TW 110114779
(71) Applicant: Ontheup Biotechnology Co., Ltd., Luodong Township, Yilan County 26561 (TW)
(72) Inventor: HO, Ta-Feng, 26561 Luodong Township, Yilan County (TW); LO, Chien-Sheng, 26561 Luodong Township, Yilan County (TW); HO, Chin-Chen, 26561 Luodong Township, Yilan County (TW)
(74) Representative: Viering, Jentschura & Partner mbB Patent- und Rechtsanwälte

(57) **Abstract**

A bone fixation device (1) is applied to a long bone. The long bone includes an epiphysis (91) and a diaphysis (92). The bone fixation device (1) includes a fixing unit (10), a stem-shaped unit (20) and a movable unit (30). The fixing unit (10) is fixed to the epiphysis (91). The stem-shaped unit (20) is implanted into the diaphysis (92). The movable unit (30) is connected to the fixing unit (10) and the stem-shaped unit (20). The movable unit (30) includes a first portion (31) and a second portion (32). One end of the first portion (31) is connected to the fixing unit (10). One end of the second portion (32) is connected to the stem-shaped unit (20). The first portion (31) and the second portion (32) are movably connected for adjustment of a relative position of the fixing unit (10) and the stem-shaped unit (20).

## Description

### BACKGROUND

### 1. Technical Field

The present disclosure relates to a bone fixation device, and more particularly, to a bone fixation device for a long bone.

### 2. Description of the Related Art

The skeletal system of the human body not only supports the body but also cooperates with the muscle system for movement and provides functions such as protecting organs. According to different functions and shapes, bones can be roughly divided into long bones, short bones, flat bones and irregular bones. Among these, long bones are mainly located in the limbs and are bones whose length is greater than their width, such as the humerus, radius, and tibia. The slender portion of a long bone is called the diaphysis or the shaft, and the enlarged portion at the two ends is called the epiphysis. The epiphysis can be combined with an articular surface of an adjacent bone to form a movable joint for performing a wide range of activities.

When the human skeleton encounters an excessive external force or impact, fracture or fragmentation often occurs. Although the human skeleton can heal itself, it is still necessary to position the bones such that they will heal in a normal state to prevent deformity or joint dysfunction in the future. Therefore, the fractured or fragmented part (i.e., the lesion) must be restored to a normal state first with the assistance of fixing members (e.g., bone nails, bone plates, or intramedullary nails) and allowed to heal.

In addition, medical treatment and reduction methods usually require appropriate treatment according to the structure of the injured bone. For example, fractures in the diaphysis are often treated with intramedullary nails; fractures near the joints or epiphysis are treated with bone nails and bone plates. However, fractures do not occur only in the epiphysis or the diaphysis. When the fracture is close to the proximal end of the diaphysis and the epiphysis, dilemma will be happened due to the limitations come from either the plate or nail system. No single solution could be the prefect answer.

### SUMMARY

In view of the above problems, a main object of the present disclosure is to provide a bone fixation device, which includes a fixing unit, a stem-shaped unit, and a movable unit. By using the movable unit to connect the fixing unit and the stem-shaped unit, the novel structure of the present disclosure can solve the problem of complicated operation steps using bone nails and bone plates in the prior art.

In order to achieve the above object, the present disclosure provides a bone fixation device, which is applied to a long bone. The long bone includes an epiphysis and a diaphysis. The bone fixation device includes a fixing unit, a stem-shaped unit and a movable unit. The fixing unit is fixed to the epiphysis. The stem-shaped unit is implanted into the diaphysis. The movable unit includes a first portion and a second portion. One end of the first portion is connected to the fixing unit. One end of the second portion is connected to the stem-shaped unit. The first portion and the second portion are movably connected for adjustment of a relative position of the fixing unit and the stem-shaped unit.

According to an embodiment of the present disclosure, the first portion includes a spherical portion, the second portion includes an accommodating groove, and the spherical portion is accommodated in the accommodating groove.

According to an embodiment of the present disclosure, the spherical portion is composed of a plurality of polygonal planes.

According to an embodiment of the present disclosure, the first portion includes a connecting rod, one end of the connecting rod is connected to the fixing unit, and the other end of the connecting rod is connected to the spherical portion.

According to an embodiment of the present disclosure, the connecting rod can move horizontally, or swing vertically relative to the second portion, with the spherical portion acting as a fulcrum, for adjustment of the relative position between the fixing unit and the stem-shaped unit.

According to an embodiment of the present disclosure, the second portion includes a limiting groove located on a side wall of the second portion and being in communication with the accommodating groove, and the connecting rod is partially accommodated in the limiting groove.

According to an embodiment of the present disclosure, the limiting groove restricts an arc of the connecting rod during horizontal movement of the connecting rod relative to the second portion.

According to an embodiment of the present disclosure, the arc of the horizontal movement is between 20 degrees and 30 degrees.

According to an embodiment of the present disclosure, a top surface of the second portion has an open end, the accommodating groove and the limiting groove are in communication with the external space through the open end, and the spherical portion and the connecting rod are disposed in the accommodating groove and the limiting groove through the open end respectively.

According to an embodiment of the present disclosure, the first portion is detachably connected to the second portion such that the fixing unit is detachably connected to the stem-shaped unit.

According to an embodiment of the present disclosure, a top surface of the stem-shaped unit is fixed to the second portion.

According to an embodiment of the present disclosure, the second portion includes a locking groove located between the open end and the accommodating groove, and the bone fixation device further includes a locking member accommodated in the locking groove to press down the spherical portion.

According to an embodiment of the present disclosure, the bone fixation device further comprises a bone fixing member disposed on the locking member, and the bone fixing member has a plurality of holes.

According to an embodiment of the present disclosure, the fixing unit has a plurality of holes.

According to an embodiment of the present disclosure, the movable unit is a universal shaft structure.

According to an embodiment of the present disclosure, each one of the first portion and the second portion has a joint, and the first portion and the second portion are pivotally connected to each other.

As described above, the bone fixation device according to the present disclosure includes a fixing unit, a stem-shaped unit, and a movable unit. The movable unit includes a first portion and a second portion, the first portion is connected to the fixing unit, and the second portion is connected to the stem-shaped unit. The fixing unit is fixed in the epiphysis and the stem-shaped unit is implanted into the diaphysis so that the surgeon can directly replace the conventional bone plate and bone nail with the bone fixation device of the present disclosure. In addition, the first portion and the second portion are movably connected such that the first portion can move horizontally, swing vertically, or rotate relative to the second portion for adjustment of the relative position between the fixing unit and the stem-shaped unit. Therefore, after fixing the fixing unit to the epiphysis and fixing the stem-shaped unit to the diaphysis, the surgeon is allowed to adjust the acceptable position between the fixing unit and the stem-shaped unit according to the alignment of the epiphysis and the diaphysis in three-dimensional space.

### BRIEF DESCRIPTION OF THE DRAWINGS

FIG. 1 illustrates a schematic view of an embodiment of a bone fixation device of the present disclosure;
FIG. 2 illustrates an explosive view of the bone fixation device shown in FIG. 1;
FIG. 3 illustrates a cross-sectional view of a movable unit shown in FIG. 2 after being assembled;
FIG. 4 illustrates a cross-sectional view of a locking member and a bone fixing member shown in FIG. 3 after being assembled;
FIG. 5 illustrates a schematic view of the bone fixation device shown in FIG. 1 applied to a long bone;
FIG. 6 illustrates a top view of the bone fixation device shown in FIG. 1;
FIG. 7 illustrates a schematic view of the fixing unit shown in FIG. 2 fixed to the epiphysis;
FIG. 8 illustrates a schematic view of the stem-shaped unit shown in FIG. 2 implanted into the diaphysis; and
FIG. 9 illustrates a schematic view of a bone fixation device according to another embodiment of the present disclosure.

### DETAILED DESCRIPTION OF THE EMBODIMENTS

In order to make the structure, characteristics, and effectiveness of the present disclosure further understood and recognized, a detailed description of the present disclosure is provided as follows, along with embodiments and accompanying figures.

First, it should be noted that for severe joint degeneration, fracture, or inflammation, joint replacement surgery using a prosthesis is usually performed. However, compared with preforming reduction and fixation surgeries to the patient's original bone, the side effects of using a prosthesis are more serious. Therefore, surgeons prefer to use bone nails and bone plates for treatment. When performing reduction surgery with only bone nails or bone plates, a bone fixation device 1 of the present disclosure can be used to solve the problems encountered in the prior art.

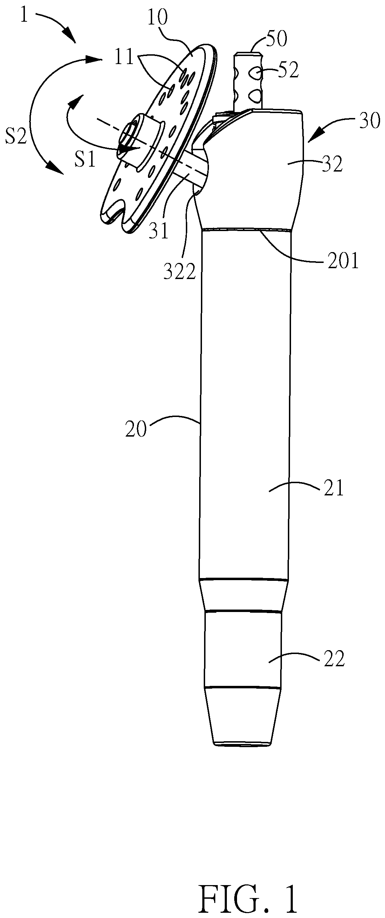

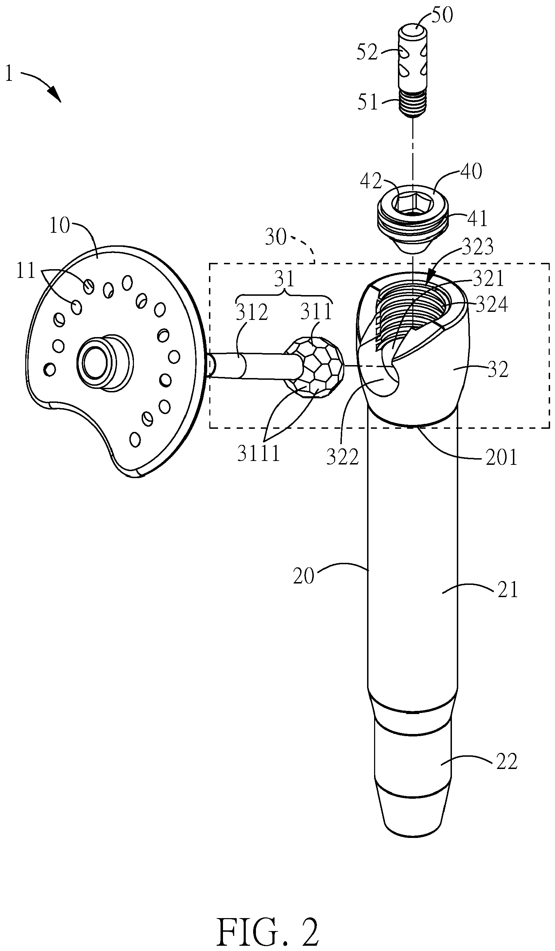

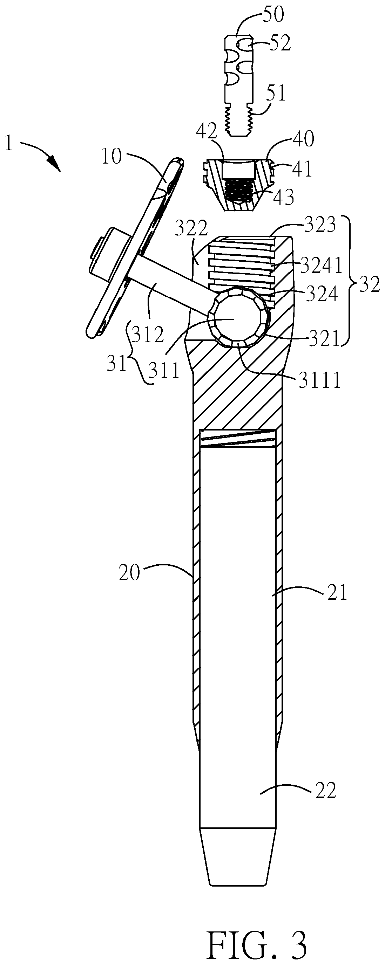

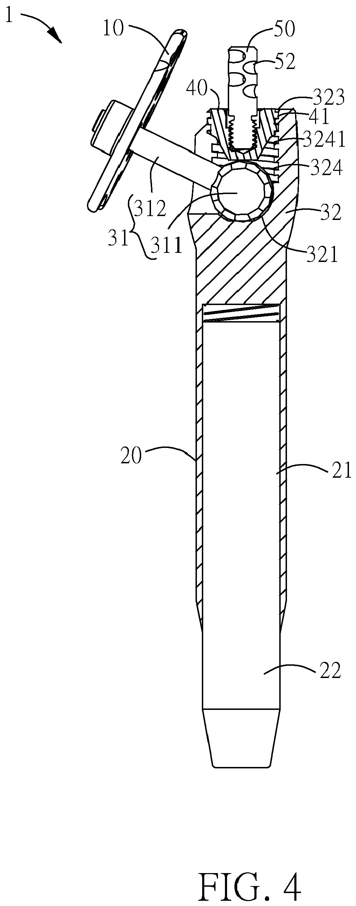

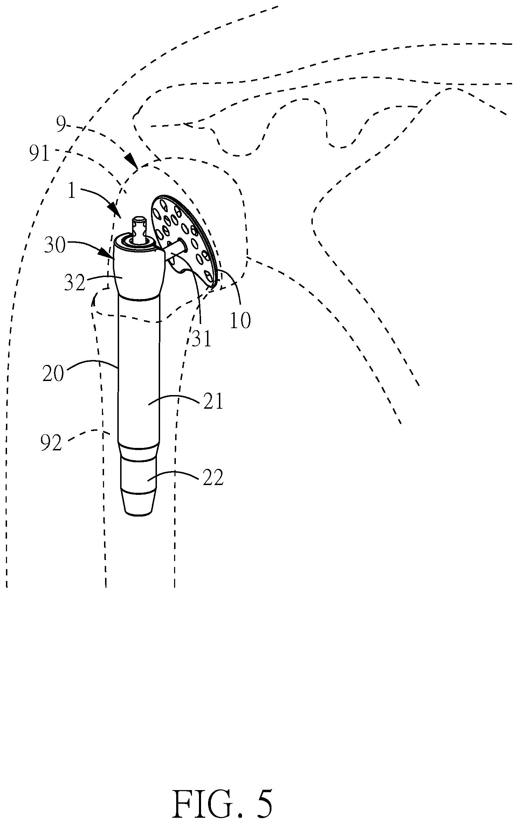

FIG. 1 illustrates a schematic view of an embodiment of a bone fixation device of the present disclosure; FIG. 2 illustrates an explosive view of the bone fixation device shown in FIG. 1; FIG. 3 illustrates a cross-sectional view of a movable unit shown in FIG. 2 after being assembled; FIG. 4 illustrates a cross-sectional view of a locking member and a bone fixing member shown in FIG. 3 after being assembled; FIG. 5 illustrates a schematic view of the bone fixation device shown in FIG. 1 applied to a long bone; please refer to the previous drawings for the following description. In this embodiment, the bone fixation device 1 is applied to a long bone 9, wherein the long bone 9 refers to a long bone having a length greater than its width and being mainly located in the limbs of the human body, such as the humerus, radius, and tibia. In FIG. 5, the humerus is presented as an example. The long bone 9 includes an epiphysis 91 and a diaphysis 92. The slender portion of the long bone 9 is the diaphysis 92, and the enlarged portion at the two ends is the epiphysis 91.

The bone fixation device 1 of this embodiment includes a fixing unit 10, a stem-shaped unit 20, and a movable unit 30. The fixing unit 10 is fixed to the epiphysis 91 and can be used to treat fractures located in the epiphysis 91. Specifically, the fixing unit 10 of this embodiment can be a plate, which can be used as a bone plate to fix the epiphysis 91. The fixing unit 10 further has a plurality of holes 11 for a bone nail (not shown) to pass through the holes 11 and to be locked into the epiphysis 91 to fix the fixing unit 10 to the epiphysis 91. In one embodiment, the fixing unit 10 may also have a plurality of holes 11 with different diameters. Among them, a hole 11 having a larger diameter can be used as a screw hole for the bone nail to pass through, and a hole 11 with a smaller diameter can be used as a suture hole for a suture to pass through so that the suture can be used to help fix the bone fragments. Among them, some of the holes having a larger diameter can be used as a screw hole for the bone to pass through, and some of the holes 11 with a smaller diameter cab be used as a suture hole for a suture to pass through so that the suture can be used to help fix the bone fragments.

In this embodiment, the stem-shaped unit 20 is implanted into the diaphysis 92. The stem-like unit 20 may be an intramedullary nail implanted into the diaphysis 92 from the proximal end of the diaphysis. Preferably, the length of the stem-shaped unit 20 can be adjusted so that the surgeon can select and use a length of the stem-shaped unit 20 appropriate for the condition of the lesion or the type of the long bone 9. For example, when the diaphysis 92 is long or there is a fracture in the middle of the diaphysis 92, a longer stem-shaped unit 20 may be selected.

In an embodiment, the stem-shaped unit 20 includes an outer sleeve 21 and a telescopic portion 22. The telescopic portion 22 is disposed in the outer sleeve 21, and the telescopic portion 22 can protrude out of the bottom of the outer sleeve 21 to expose part of its structure. In addition, the telescopic portion 22 can be moved relative to the outer sleeve 21 for adjustment of the length of the telescopic portion 22 which is exposed from the bottom of the outer sleeve 21 such that the overall length of the stem-shaped unit 20 is adjusted. In another embodiment, medical device manufacturers can also provide stem-shaped units 20 of different lengths for surgeons to choose. In other words, the present disclosure does not limit the length of the stem-shaped unit 20.

The bone fixation device 1 of this embodiment is connected to the fixing unit 10 and the stem-shaped unit 20 by the movable unit 30. The movable unit 30 includes a first portion 31 and a second portion 32. One end of the first portion 31 is connected to the fixing unit 10, and one end of the second portion 32 is connected to the stem-shaped unit 20. Preferably, a top surface 201 of the stem-shaped unit 20 is fixed to the second portion 32. For example, the second portion 32 and the stem-shaped unit 20 may be integrally formed.

Additionally, the first portion 31 and the second portion 32 are movably connected to each other. For example, the first portion 31 and the second portion 32 can be pivotally connected to each other or connected to each other in a pivot-like manner such that the first portion 31 can swing or move horizontally relative to the second portion 32 for adjustment of the relative position between the fixing unit 10 and the stem-shaped unit 20. In other words, the first portion 31 and the second portion 32 are interconnected in nearly universal way for adjusting an acceptable position of the fixing unit 10 and the stem-shaped unit 20.

In this embodiment, the first portion 31 and the second portion 32 are connected to each other in a pivot-like manner. Specifically, the first portion 31 of the present embodiment includes a spherical portion 311, the second portion 32 includes an accommodating groove 321, as shown in FIG. 3 and FIG. 4, and the spherical portion 311 is accommodated in the accommodating groove 321. Preferably, the accommodating groove 321 is an arc-shaped groove so as to facilitate the rotation of the spherical portion 311 in the accommodating groove 321. Since the spherical portion 311 can be rotated in the accommodating groove 321, the first portion 31 and the second portion 32 are movably connected for adjustment of the relative position between the fixing unit 10 and the stem-shaped unit 20.

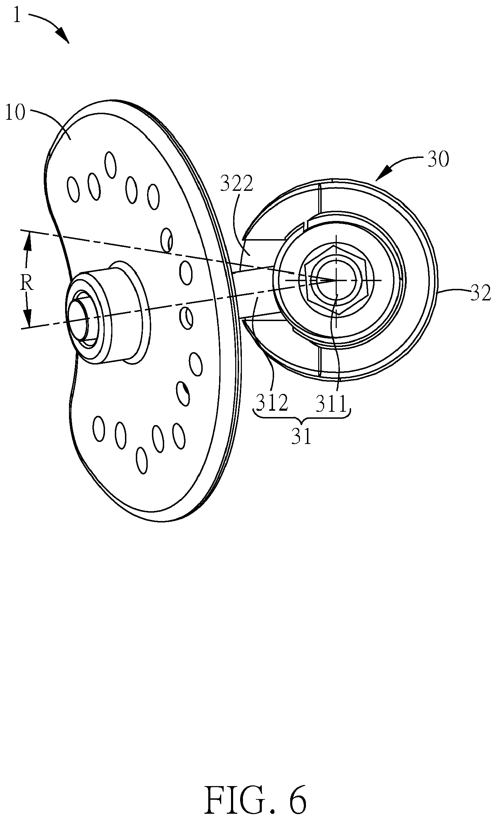

Specifically, the first portion 31 further includes a connecting rod 312, wherein one end of the connecting rod 312 is connected to the fixing unit 10 and the other end of the connecting rod 312 is connected to the spherical portion 311. Therefore, the spherical portion 311 acts as a fulcrum for the connecting rod 312 to move horizontally S1, swing vertically S2 (as shown in FIG. 1), or rotate relative to the second portion 32. When the connecting rod 312 moves horizontally S1 to either side or swings vertically S2 relative to the second portion 32, the fixing unit 10, being connected to the connecting rod, is driven by the connecting rod 312 also to move horizontally S1 to either side or swing vertically S2 relative to the second portion 32 such that the relative position between the fixing unit 10 and the stem-shaped unit 20 can be adjusted. Please also refer to FIG. 6, which illustrates the connecting rod 312 moving horizontally S1 to either side relative to the second portion 32, with the spherical portion 311 acting as a fulcrum. FIG. 6 is a top view of the bone fixation device shown in FIG. 1. FIG. 6 shows an arc R of the movement of the connecting rod 312 to either side of the second portion 32, which will be further described later.

In addition, the connecting rod 312 can rotate relative to the second portion 32 with the spherical portion 311 acting as the fulcrum, which means that the spherical portion 311 as an axis such that the connecting rod 312 can rotate relative to the second portion 32. At the same time, the connecting rod 312 drives the fixing unit 10 to rotate relative to the second portion 32 for adjustment of the relative position between the fixing unit 10 and the stem-shaped unit 20.

It should be noted that in the aforementioned embodiment, the second portion 32 and the connected stem-shaped unit 20 are regarded as fixed parts, and the first portion 31 and the connected fixing unit 10 are regarded as the moving parts. In addition, the connecting rod 312 can also rotate relative to the second portion 32 with the spherical portion 311 acting as the axis, and the connecting rod 312 drives the fixing unit 10 relative to the second portion 32 at the same time, for adjustment of the relative position between the fixing unit 10 and the stem-shaped unit 20.

On the other hand, if the first portion 31 and its connected fixing unit 10 are regarded as fixed parts and the second portion 32 and its connected stem-shaped unit 20 are regarded as moving parts, then by operating the stem-shaped unit 20, the accommodating groove 321 of the second portion 32 can be moved horizontally or swung vertically relative to the spherical portion 311. Therefore, with the design of the movable unit 30, the fixing unit 10 can move horizontally, swing vertically, or rotate relative to the stem-shaped unit 20; alternatively, the stem-shaped unit 20 can move horizontally, swing vertically, or rotate relative to the fixing unit 10. However, for ease of understanding, in this embodiment, the operation of the fixing unit 10 and the first portion 31 moving horizontally S1, swinging vertically S2, or rotating relative to the second portion 32 (or the stem-shaped unit 20) is mainly described as an example.

Preferably, the second portion 32 of this embodiment includes a limiting groove 322. The limiting groove 322 is located on a side wall of the second portion 32 and is in communication with the accommodating groove 321 such that a portion of the connecting rod 312 can be accommodated in the limiting groove 322. Specifically, the connecting rod 312 passes through the limiting groove 322 such that the spherical portion 311 is located in the accommodating groove 321 and the fixing unit 10 is located outside the accommodating groove 321. Since the connecting rod 312 is partially accommodated in the limiting groove 322, the limiting groove 322 can restrict the arc R of the connecting rod 312 during horizontal movement relative to the second portion 32, as shown in FIG. 6.

From the viewing angle of FIG. 6, relative to the fixing unit 10, the spherical portion 311 can be regarded as the center of the circle and the connecting rod 312 as the radius. From this perspective, the fixing unit 10 can move horizontally in either direction relative to the second portion 32, forming an arc trajectory. The design of the limiting groove 322 can restrict the range of movement of the connecting rod 312, thereby limiting the arc R of the arc trajectory formed by the fixing unit 10. Preferably, the arc R can be between 20 degrees and 30 degrees. In other words, the limiting groove 322 can restrict the arc R of the horizontal movement of the fixing unit 10 to between 20 degrees and 30 degrees.

In addition, the bottom edge of the limiting groove 322 can restrict the extent of the downward vertical movement of the connecting rod 312, thereby restricting the downward vertical movement of the fixing unit 10. In other embodiments, a convex portion can be formed on the inner wall of the limiting groove 322 and above the connecting rod 312 to restrict the extent of the upward vertical movement of the connecting rod 312 and the fixing unit 10.

In this embodiment, a top surface of the second portion 32 has an open end 323, and the accommodating groove 321 and the limiting groove 322 are in communication with the outside through the open end 323. In other words, the top surface of the second portion 32 is the open end 323, and both the accommodating groove 321 and the limiting groove 322 are in communication with the open end 323 and are further in communication with the space outside (the external space) of the second portion 32. The spherical portion 311 of the first portion 31 and the connecting rod 312 of the first portion 31 can be disposed in the accommodating groove 321 and the limiting groove 322 through the open end 323 of the second portion 32 respectively such that the fixing unit 10 and the first portion 31 are assembled to the second portion 32 and the stem-shaped unit 20. In other words, the first portion 31 of this embodiment is detachably connected to the second portion 32, so the fixing unit 10 is detachably connected to the stem-shaped unit 20.

The second portion 32 of this embodiment further includes a locking groove 324, which is located between the open end 323 and the accommodating groove 321. In other words, the second portion 32 has the accommodating groove 321 and the locking groove 324 disposed therein, the accommodating groove 321 is located below and close to the stem-shaped unit 20, and the locking groove 324 is located above and close to the open end 323. The locking groove 324 has an inner thread 3241. Correspondingly, the bone fixation device 1 further includes a locking member 40, and the locking member 40 has an outer thread 41, which corresponds to the inner thread 3241 of the locking groove 324. In addition, the locking member 40 of the present embodiment further has disposed therein a lock hole 42, such as a polygonal lock hole 42 suitable for wrench operation.

After the relative position between the fixing unit 10 and the stem-shaped unit 20 is adjusted to an appropriate position, the locking member 40 can be accommodated in the locking groove 324, with the outer thread 41 and the inner thread 3241 corresponding to each other. Next, the locking member 40 can be screwed downward with a wrench such that the locking member 40 presses down against the spherical portion 311 of the first portion 31, thereby fixing the relative position between the fixing unit 10 and the stem-shaped unit 20.

Preferably, the spherical portion 311 is composed of a plurality of polygonal planes 3111, as shown in FIG. 2. In other words, the spherical portion 311 of the present embodiment is preferably a polyhedral sphere, which can increase the frictional force between the spherical portion 311 and the accommodating groove 321. The polygonal planes 3111 may be the same or different, which is not limited in the present disclosure. For example, the spherical portion 311 may be composed of a plurality of pentagonal planes. Alternatively, the spherical portion 311 may be composed of a plurality of triangular planes, quadrilateral planes, and pentagonal planes. After the relative position between the fixing unit 10 and the stem-shaped unit 20 is adjusted to the aligned angle, if the spherical portion 311 comprises a smooth spherical surface and the accommodating groove 321 is a smooth arc-shaped groove, the spherical portion 311 and the accommodating groove 321 can easily be shifted with respect to each other due to uneven application of force or accidental contact of the surgeon, thereby changing the relative position between the fixing unit 10 and the stem-shaped unit 20. The spherical portion 311 formed of polygonal planes 3111 is used to increase the frictional force between the spherical portion 311 and the accommodating groove 321 so as to prevent the first portion 31 from moving relative to the second portion 32 and to fix the relative position between the fixing unit 10 and the stem-shaped unit 20. In other embodiments, the inner surface of the accommodating groove 321 may also be composed of a plurality of polygonal planes, which can also facilitate proper positioning.

Preferably, the bone fixation device 1 further includes a bone fixing member 50, which is disposed on the locking member 40. For example, the locking member 40 can be screwed to the locking groove 324 with a wrench, and after the wrench is removed, the bone fixing member 50 can be disposed in the locking hole 42 so that the bone fixing member 50 is now disposed on the locking member 40. Specifically, the locking member 40 can further include an inner thread 43 located at the lower edge of the polygonal lock hole 42. Correspondingly, the bone fixing member 50 has an outer thread 51, which corresponds to the inner thread 43 of the locking member 40. The bone fixing member 50 is disposed in the lock hole 42 of the locking member 40 with the end having the outer thread 51 inserted therein, and the bone fixing member 50 is fixed to the locking member 40 by screwing the outer thread 51 into the inner thread 43.

Furthermore, the bone fixing member 50 has a plurality of holes 52 located above the outer thread 51. When the bone fixing member 50 is disposed on the locking member 40, the hole 52 is located outside the locking member 40. The hole 52 is provided for sutures to pass through to help fix the fragmented bones.

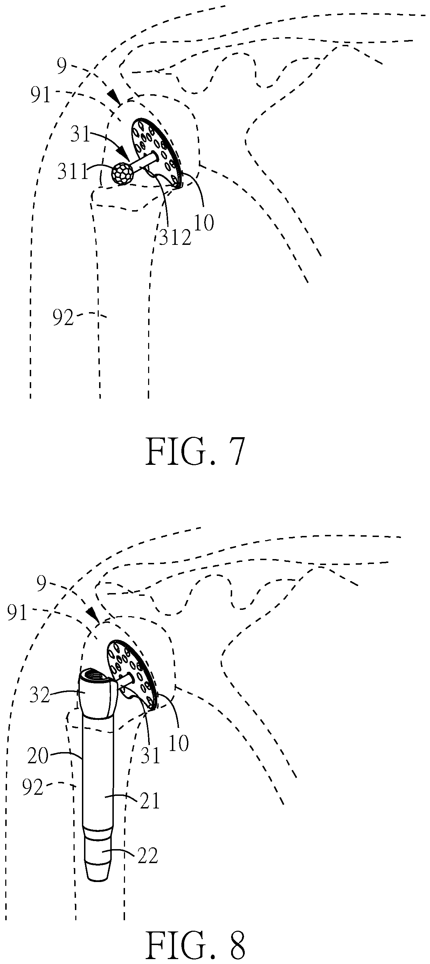

FIG. 7 illustrates a schematic view of the fixing unit shown in FIG. 2 fixed to the epiphysis, and FIG. 8 illustrates a schematic view of the stem-shaped unit shown in FIG. 2 implanted in the diaphysis. Please refer to FIG. 7 and FIG. 8 for the assembling method of the bone fixation device 1 of the present embodiment. If the fracture site is at the proximal end of the diaphysis 92 or at the epiphysis 91, then after the fracture is located, the surgeon must first slightly lift the epiphysis 91 to separate the epiphysis 91 from the diaphysis 92. Next, the surgeon can pass a bone nail through the hole 11 to fix the fixing unit 10 on the epiphysis 91. If the fracture is very serious, the surgeon can also pass sutures through the hole 11 to fix the bone fragments. Since the first portion 31 is connected to the fixing unit 10, the first portion 31 and the fixing unit 10 are located near the epiphysis 91, as shown in FIG. 7.

Next, the surgeon implants the stem-shaped unit 20 into the backbone diaphysis 92 and causes the second portion 32 on the top surface 201 of the stem-shaped unit 20 to protrude out of the diaphysis 92. Depending on the fracture condition of the diaphysis 92, the surgeon can also adjust the stem-shaped unit 20 to an appropriate length by moving the telescopic portion 22. For example, the telescopic portion 22 can be pulled from the outer sleeve 21 to increase the overall length of the stem-shaped unit 20. After the stem-shaped unit 20 is implanted into the diaphysis 92, the second portion 32 must be protruded out of the diaphysis 92 so that the second portion 32 can be assembled to the first portion 31.

Specifically, the open end 323 of the second portion 32 is aligned with the spherical portion 311 of the first portion 31 and the connecting rod 312 such that the spherical portion 311 and the connecting rod 312 are disposed in the accommodating groove 321 and the limiting groove 322 respectively, thereby assembling the fixing unit 10 and the first portion 31 with the second portion 32 and the stem-shaped unit 20, as shown in the FIG. 8. At this time, the surgeon can adjust the relative position between the fixing unit 10 and the stem-shaped unit 20 according to the position of the lesion. For example, the surgeon may move the patient's arm, thereby moving the stem-shaped unit 20 and the connected second portion 32 and shifting the spherical portion 311 within the accommodating groove 321. Correspondingly, the connecting rod 312 of the first portion 31 can, with respect to the second portion 32, move horizontally, swing vertically, or rotate with the spherical portion 311 acting as the axis for adjustment of the relative position between the fixing unit 10 and the stem-shaped unit 20.

After the relative position between the fixing unit 10 and the stem-shaped unit 20 is adjusted to a suitable position, such as a position suitable for the alignment of the epiphysis 91 and the diaphysis 92, then the locking member 40 is disposed in the locking groove 324. For example, the locking member 40 may be screwed downward with a wrench press against the spherical portion 311 of the first portion 31, thereby fixing the relative position between the fixing unit 10 and the stem-shaped unit 20. Finally, the wrench is removed and the bone fixing member 50 is disposed in the lock hole 42 to complete the assembly of the bone fixation device 1 of this embodiment, as shown in FIG. 5.

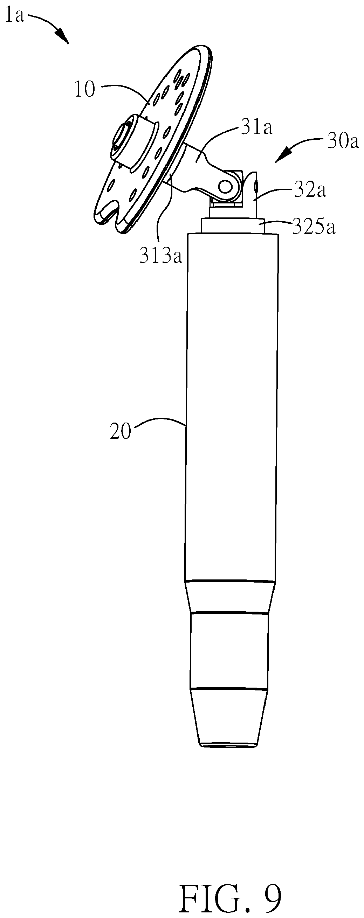

FIG. 9 is a schematic view of a bone fixation device according to another embodiment of the present disclosure. Please refer to FIG. 9. A bone fixation device 1a of this embodiment includes the fixing unit 10, the stem-shaped unit 20, and a movable unit 30a. The configurations of the fixing unit 10 and the stem-shaped unit 20 are the same as those in the previous embodiment, so the same reference numerals are used. The movable unit 30a of this embodiment is a universal shaft structure and also includes a first portion 31a and a second portion 32a. One end of the first portion 31a has a joint 313a connected to the fixing unit 10. One end of the second portion 32a also has a joint 325a connected to the stem-shaped unit 20.

In addition, the first portion 31a and the second portion 32a are pivotally connected to each other such that the first portion 31a and the second portion 32a are movably connected for adjustment of the relative position between the fixing unit 10 and the stem-shaped unit 20. For example, the fixing unit 10 can move horizontally or swing vertically relative to the stem-shaped unit 20.

As described above, the bone fixation device according to the present disclosure includes a fixing unit, a stem-shaped unit, and a movable unit. The movable unit includes a first portion and a second portion, the first portion is connected to the fixing unit, and the second portion is connected to the stem-shaped unit. The fixing unit is fixed in the epiphysis and the stem-shaped unit is implanted into the diaphysis so that the surgeon can directly replace the conventional bone plate and bone nail with the bone fixation device of the present disclosure. In addition, the first portion and the second portion are movably connected such that the first portion can move horizontally, swing vertically, or rotate relative to the second portion for adjustment of the relative position between the fixing unit and the stem-shaped unit. Therefore, after fixing the fixing unit to the epiphysis and fixing the stem-shaped unit to the diaphysis, the surgeon is allowed to still adjust the acceptable positions of the fixing unit and the stem-shaped unit according to the alignment of the epiphysis and the diaphysis in three-dimensional space.

It is noted that the above-described embodiments are merely illustrative of preferred embodiments of the present disclosure, and that in order to prevent redundancy, not all possible combinations of variations are described in detail; various changes and modifications may be made to the described embodiments without departing from the scope of the disclosure as described by the appended claims.

## Claims

1. A bone fixation device (1) applied to a long bone, which includes an epiphysis (91) and a diaphysis (92), the bone fixation device (1) comprising:
a fixing unit (10) fixed to the epiphysis (91);
a stem-shaped unit (20) implanted into the diaphysis (92); and
a movable unit (30) connected to the fixing unit (10) and the stem-shaped unit (20), the movable unit (30) comprising:
a first portion (31) having one end connected to the fixing unit (10); and
a second portion (32) having one end connected to the stem-shaped unit (20), the first portion (31) and the second portion (32) being movably connected for adjustment of a relative position of the fixing unit (10) and the stem-shaped unit (20).

2. The bone fixation device (1) as claimed in Claim 1, wherein the first portion (31) includes a spherical portion (311), the second portion (32) includes an accommodating groove (321), and the spherical portion (311) is accommodated in the accommodating groove (321).

3. The bone fixation device (1) as claimed in Claim 2, wherein the spherical portion (311) is composed of a plurality of polygonal planes (3111).

4. The bone fixation device (1) as claimed in Claim 2, wherein the first portion (31) includes a connecting rod (312), one end of the connecting rod (312) is connected to the fixing unit (10), and the other end of the connecting rod (312) is connected to the spherical portion (311).

5. The bone fixation device (1) as claimed in Claim 4, wherein the connecting rod (312) moves horizontally or swings vertically relative to the second portion (32), with the spherical portion (311) acting as a fulcrum, for adjustment of the relative position between the fixing unit (10) and the stem-shaped unit (20).

6. The bone fixation device (1) as claimed in Claim 5, wherein the second portion (32) includes a limiting groove (322) located on a side wall of the second portion (32) and being in communication with the accommodating groove (321), and the connecting rod (312) is partially accommodated in the limiting groove (322).

7. The bone fixation device (1) as claimed in Claim 6, wherein a top surface of the second portion (32) has an open end (323), the accommodating groove (321) and the limiting groove (322) are in communication with the external space through the open end (323), and the spherical portion (311) and the connecting rod (312) are disposed in the accommodating groove (321) and the limiting groove (322) through the open end (323) respectively.

8. The bone fixation device (1) as claimed in Claim 7, wherein the first portion (31) is detachably connected to the second portion (32) such that the fixing unit (10) is detachably connected to the stem-shaped unit (20).

9. The bone fixation device (1) as claimed in Claim 7, wherein a top surface (201) of the stem-shaped unit (20) is fixed to the second portion (32).

10. The bone fixation device (1) as claimed in Claim 7, wherein the second portion (32) includes a locking groove (324) located between the open end (323) and the accommodating groove (321), and the bone fixation device (1) further includes a locking member (40) accommodated in the locking groove (324) to press down on the spherical portion (311).

11. The bone fixation device (1) as claimed in Claim 10 further comprising a bone fixing member (50) disposed on the locking member (40), wherein the bone fixing member (50) has a plurality of holes (52).

12. The bone fixation (1) device as claimed in Claim 1, wherein the fixing unit (10) has a plurality of holes (11).

13. The bone fixation device (1a) as claimed in Claim 1, wherein the movable unit (30a) is a universal shaft structure.

14. The bone fixation device (1a) as claimed in Claim 13, wherein each one of the first portion (31a) and the second portion (32a) has a joint (313a or 325a), and the first portion (31a) and the second portion (32a) are pivotally connected to each other.
